# EUROPÄISCHE PATENTSCHRIFT

(11) **EP 2 996 625 B1**
(45) Veröffentlichungstag und Bekanntmachung des Hinweises auf die Patenterteilung: **09.01.2019**
(21) Anmeldenummer: 14711699.0
(22) Anmeldetag: 28.02.2014
(51) Int. Cl.: A61C 9/00, G06K 17/00, G06K 19/00, A61B 90/00

(54) **VERFAHREN UND VORRICHTUNG ZUR STEUERUNG EINES COMPUTERPROGRAMMS MITTELS EINES INTRAORALEN SCANNERS**
METHOD AND DEVICE FOR CONTROLLING A COMPUTER PROGRAM BY MEANS OF AN INTRAORAL SCANNER
PROCÉDÉ ET DISPOSITIF DE COMMANDE D'UN PROGRAMME INFORMATIQUE AU MOYEN D'UN SCANNER INTRAORAL

(30) Priorität: 28.02.2013 DE 102013203449
(43) Veröffentlichungstag der Anmeldung: 23.03.2016
(73) Patentinhaber: Sirona Dental Systems GmbH, 64625 Bensheim (DE)
(72) Erfinder: SCHNEIDER, Sascha, 64367 Mühltal (DE)
(74) Vertreter: Sommer, Peter
(86) Internationale Anmeldenummer: PCT/EP2014/053903
(87) Internationale Veröffentlichungsnummer: WO 2014/131866

(56) Entgegenhaltungen:
- EP-A1- 2 301 330
- DE-A1- 19 534 998
- US-A1- 2007 092 854
- US-A1- 2008 153 067
- US-A1- 2009 317 757

## Beschreibung

### Technisches Gebiet

Die vorliegende Erfindung betrifft ein Verfahren zur Steuerung eines Computerprogramms mittels eines intraoralen Scanners.

### Stand der Technik

Intraorale Scansysteme ermöglichen es einem Zahnarzt, mittels eines fließenden Aufnahmeverfahrens oder durch Aufnahme von wenigen Einzelbildern dreidimensionale Darstellungen des Mundinnenraums zu erstellen. Hierzu wird der Kopf einer Handkamera in geringem Abstand über aufzunehmende Zähne geführt. Aus den mittels der Kamera erfassten Daten kann mittels einer CAD/CAM-Software (Computer Aided Design/Computer Aided Manufacturing) ein dreidimensionales Modell erstellt werden. Dieses kann anschließend als Grundlage für eine Rekonstruktion dienen.

Bei der Verwendung solcher intraoraler Scansysteme hat der ausführende Zahnarzt typischerweise die intraorale Scankamera in der Hand, trägt Schutzhandschuhe und kommt mit dem Mundinnenraum des Patienten in Kontakt, so dass die Schutzhandschuhe mit Speichel oder Blut benetzt werden. Wenn er gleichzeitig die CAD/CAM-Software bedienen möchte, beschmutzt er mit seinen Händen Maus, Tastatur oder Touchscreen-Bildschirm des die Software ausführenden Computers und kann von dort gegebenenfalls Keime in den Patientenmund bringen. Verfahren zur kontaktlosen Steuerung einer CAD/CAM-Software, welche keine Steuerung über ein Eingabe-Interface am zugehörigen PC System benötigen, sind nicht bekannt. Eine Sprachsteuerung ist für derartige Systeme nicht verfügbar, da diese aufgrund des vom Zahnarzt getragenen Mundschutzes und von in der Zahnarztpraxis nicht vermeidbaren Umgebungsgeräuschen störanfällig ist und gegebenenfalls erst eingelernt werden müsste.

Die Platzierung von Markern im Mundraum eines Patienten ist bekannt. In der der EP 2 301 330 A1 wird ein Gebisselement beschrieben, das eine Kodierung, beispielsweise in Form eines maschinenlesbaren Barcodes, aufweist. Die US 2007/0092854 A1 beschreibt ein Dentalimplantat, welches mit Markern versehen ist. Aus der DE 195 34 998 A1 ist ein Zahnersatz bekannt, der mit einem Marker in Form eines Barcodes, eines alphanumerischen Zeichens oder eines Logos versehen ist.

Es ist die Aufgabe der vorliegenden Erfindung, eine kontaktlose Steuerung einer Software im dentalmedizinischen Bereich zu ermöglichen, bei der der Zahnarzt die Kamera oder einen anderen Scanner nicht aus der Hand legen muss.

### Darstellung der Erfindung

Diese Aufgabe wird durch das erfindungsgemäße Verfahren zur Steuerung eines Computerprogramms gelöst, worin bei Erfassung eines optischen Markers durch einen intraoralen Scanner ein Steuersignal an das Computerprogramm gesendet wird, welches das Computerprogramm in einen vorgegebenen Zustand schaltet. Der Scanner ist insbesondere eine 2D-Kamera oder ein 3D-Scanner. Das Computerprogramm ist ein CAD/CAM-Programm.

Der vorgegebene Zustand ist ausgewählt aus der Gruppe bestehend aus einem Wechsel eines Bildfeldes, dem Löschen eines Bildbereiches, einer Bildberechnung und einer Restaurationsberechnung. Hierdurch ist es dem Zahnarzt möglich, ohne den intraoralen Scanner aus der Hand zu legen, die Funktionen des CAD/CAM-Programms zu steuern. So kann er beispielsweise bei einer Bildaufnahme das Bildfeld wechseln, indem er den intraoralen Scanner auf einen diesem Zustand zugeordneten Marker richtet. Auch ist es ihm so möglich, zwischen dem Erfassen eines Bildfeldes und dem Löschen eines Bildbereiches zu wechseln. Eine dreidimensionale Bildberechnung oder eine Zahnrestaurationsberechnung kann auf diese Weise ebenfalls gestartet werden. Hierzu ist bevorzugt, dass der optische Marker auf einem Träger aufgebracht ist, der mindestens einen optischen Marker aufweist. Dem Zahnarzt kann so bei seiner Arbeit, beispielsweise eine Tafel mit optischen Markern für alle vorgegebenen Zustände vorliegen, so dass er diese schalten kann, indem er den intraoralen Scanner auf den dem jeweiligen Zustand zugeordneten Marker richtet.

Der optische Marker ist bevorzugt ein zweidimensionaler Barcode. Dieser kann von der Software selbst erzeugt werden oder importiert werden. Es ist auch möglich den Barcode aufgrund eines fremdgenerierten Etiketts anzulernen, indem dieses einmalig mit dem intraoralen Scanner gescannt wird.

Weiterhin ist es erfindungsgemäß möglich, dass der optische Marker auf einen Scanbody aufgebracht ist oder aus einem Scanbody besteht. Hierbei kann es sich beispielsweise um einen mit einem Binärcode versehenen Scanbody handeln, wie er in der US 2008/0233537 A1 beschrieben wird. Um den Typ eines in einem Kiefer befindlichen Implantates zu bestimmen, muss der Nutzer bei herkömmlichen CAD/CAM-Scans des entsprechenden Areals in der Software den passenden Typ des Implantates manuell auswählen. Um die Position eines Implantates zu bestimmen, kommen in der Regel Algorithmen zum Einsatz, die in den Scandaten bestimmte dreidimensionale Strukturen eines aufgesteckten Scanbodys suchen und darüber dann die Position und Orientierung des darunterliegenden Implantates bestimmen. Wenn der Scanbody beispielsweise aufgrund seiner Farbe und/oder Musterung als optischer Marker verwendet wird, kann dadurch erfindungsgemäß automatisch der Typ des darunter befindlichen Implantates bestimmt werden. Es ist auch möglich, einen optischen Marker auf der Oberfläche des Scanbodys aufzubringen, beispielsweise in Form eines zweidimensionalen Barcodes. Neben der Schaltung des Computerprogramms in einen Zustand, in dem das dem Scanbody entsprechende Implantat in den erfassten Datensatz eingefügt wird, ist es erfindungsgemäß beispielsweise auch möglich, das Computerprogramm in einen Zustand zu schalten, in dem ein Scan mit einer anderen, vorzugsweise höheren Auflösung erfolgt als in anderen Mundarealen. Weiterhin ist es möglich, das Computerprogramm auf diese Weise in einen Konstruktionsmodus zu schalten oder eine Vorschlagsberechnung zu starten. Eine solche Vorschlagsberechnung ermöglicht insbesondere eine automatische Generierung eines Einzelzahn-Abutments in reduzierter Form, eines Einzelzahn-Abutments in Vollkontur, eines Pfostenelements für einen Steg oder eine verschraubte Brücke, oder eine Entscheidung über eine einschichtige oder mehrschichtige Versorgung. Der Scanbody kann auch auf eine Gingiva aufgebracht werden, und das Computerprogramm bei Erfassung des Scanbodys oder eines optischen Markers auf dem Scanbody in einen Zustand geschaltet werden, in dem eine Bohrstelle in einer im Anschluss an einen Modellscan zu konstruierenden Bohrschablone markiert oder definiert wird. Das erfindungsgemäße Verfahren ermöglicht somit die Verwendung eines intraoralen Scanners zur Steuerung eines Computerprogramms.

Ein Computerprogramm, welches als CAD/CAM-Programm ausgeführt ist, ist mittels des erfindungsgemäßen Verfahrens steuerbar. Vorzugsweise ist das Computerprogramm dazu eingerichtet, mindestens einen optischen Marker auf einem Monitor darzustellen, wobei der optische Marker eine Beschriftung aufweist, die einen Zustand des Computerprogramms angibt, in welchen das Computerprogramm durch Erfassung eines optischen Markers durch einen intraoralen Scanner geschaltet werden kann. Dadurch ist es möglich, die optischen Marker zur Steuerung des Computerprogramms, beispielsweise auf dem Monitor eines CAD/CAM-Systems, darzustellen, so dass der Zahnarzt sie dort mit dem intraoralen Scanner erfassen kann. Alternativ ist auch die Darstellung auf einem separaten System, wie beispielsweise einem Tablet PC möglich. Die Beschriftung zeigt dem Zahnarzt mittels eines Textes oder einer Grafik, in welchen Zustand das Computerprogramm geschaltet wird, wenn er den intraoralen Scanner über den entsprechenden optischen Marker führt. Im Gegensatz zur Bereitstellung der optischen Marker auf einem analogen Medium ist es hierbei möglich, eine Menüstruktur zu schaffen, so dass durch Anwählen eines optischen Markers einige optische Marker ausgeblendet und andere optische Marker eingeblendet werden, so dass die optischen Marker zur Steuerung des Computerprogramms dem Zahnarzt in übersichtlicher Weise präsentiert werden können.

Eine Vorrichtung zur Steuerung des Computerprogramms, umfasst einen Träger, auf dem mindestens ein optischer Marker angeordnet ist. Bei dem Träger kann es sich beispielsweise um einen laminierten Begleitzettel handeln. Jeder optische Marker weist dabei eine Beschriftung auf, die einen Zustand des Computerprogramms angibt, in welchen das Computerprogramm durch Erfassung eines optischen Markers durch einen intraoralen Scanner geschaltet werden kann. Der optische Marker ist hierbei vorzugsweise ein zweidimensionaler Barcode.

Ein intraoraler Scanner und ein Rechengerät, auf dem das Computerprogramm abläuft, können als Set bereitgestellt werden. Bevorzugt umfasst dieses Set weiterhin die Vorrichtung zur Steuerung des Computerprogramms.

Eine Vorrichtung welche einen intraoralen Scanner umfasst kann dazu ausgebildet sein, bei Erfassung eines optischen Markers durch den intraoralen Scanner ein Steuersignal zu erzeugen.

### Kurze Beschreibung der Zeichnungen

Ausführungsbeispiele der Erfindung sind in den Zeichnungen dargestellt und in der folgenden Beschreibung näher erläutert.
In Fig. 1 sind eine intraorale Kamera und eine Vorrichtung zur Steuerung eines Computerprogramms zur Verwendung in einer Ausführungsform des erfindungsgemäßen Verfahrens dargestellt.
Fig. 2 zeigt mehrere zweidimensionale Barcodes, die von einem Computerprogramm gemäß einer Ausführungsform der Erfindung auf einem Monitor generiert werden.
Fig. 3 zeigt die Anordnung eines Scanbodys in einem Kiefer in einer Ausführungsform der Erfindung.

### Ausführungsbeispiele

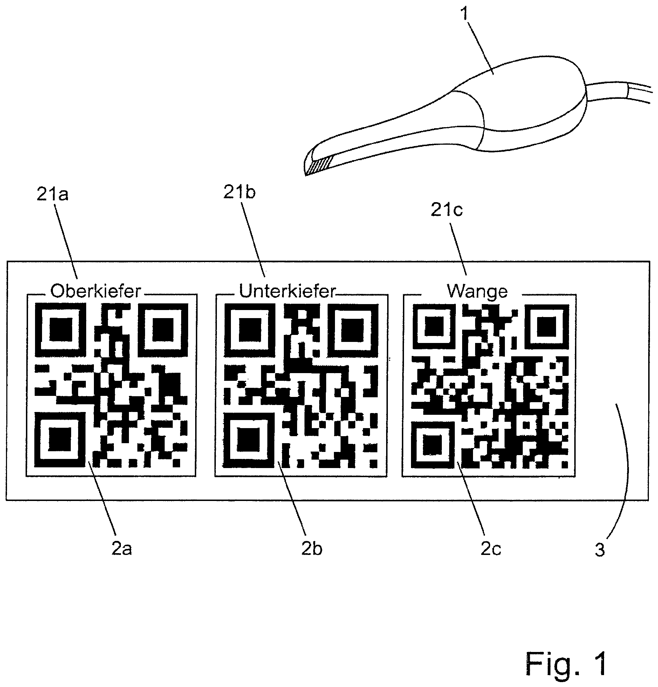

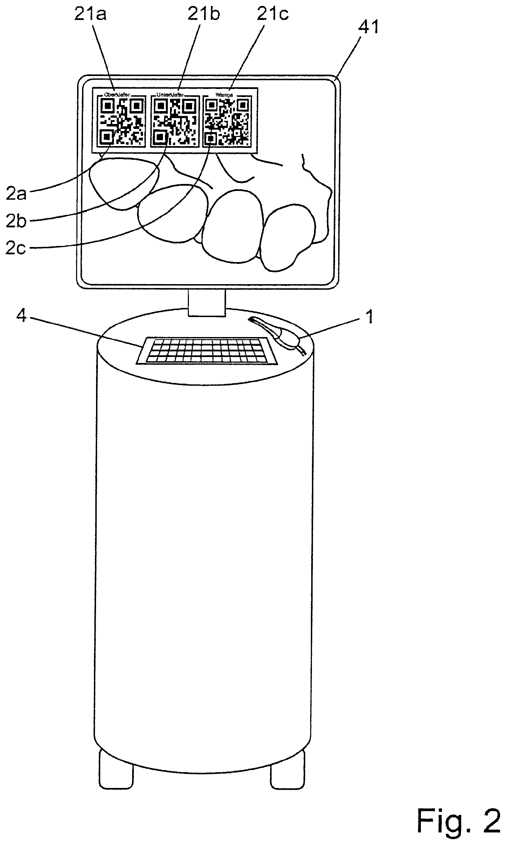

In einer Ausführungsform der Erfindung wird ein CAD/CAM-Programm auf einer Vorrichtung zur digitalen Abformung und Herstellung von Zahnrestaurationen (beispielsweise CEREC AC + MC XL der Anmelderin) ausgeführt. Als Scansystem zur digitalen Abformung dient eine intraorale 3D-Kamera 1, die in Fig. 1 dargestellt ist. Das CAD/CAM-Programm ist dazu eingerichtet, zwischen unterschiedlichen Zuständen, wie beispielsweise einer Erfassung eines Bildfeldes, dem Löschen eines Bildbereiches, einer Bildberechnung und einer Restaurationsberechnung zu schalten, wenn die intraorale Kamera 1 einen optischen Marker in Form eines zweidimensionalen Barcodes 2a, 2b, 2c erfasst. Jeweils ein zweidimensionaler Barcode 2a, 2b, 2c ist auf einem laminierten Zettel 3 aufgebracht, um jeweils einen Schaltzustand auszulösen. Jeder zweidimensionale Barcode 2a, 2b, 2c weist dabei eine Beschriftung 21a, 21b, 21c auf, aus welcher ein Zahnarzt erkennen kann, welchem Schaltvorgang der jeweilige zweidimensionale Barcode 2a, 2b, 2c zugeordnet ist. Der laminierte Zettel 3 dient als Vorrichtung zur Steuerung des CAD/CAM-Programms und kann vom Zahnarzt während einer digitalen Abformung auf einer Arbeitsfläche abgelegt werden oder am Monitor des CAD/CAM-Systems angebracht werden. Das CAD/CAM-Programm ist weiterhin dazu eingerichtet, die zweidimensionalen Barcodes 2a, 2b, 2c zur Schaltung der vorgegebenen Zustände auf einem Monitor 41 eines CAD/CAM-Systems 4 zusammen mit der Beschriftung 21a, 21b, 21c darzustellen. Dies ist in Fig. 2 dargestellt. Das CAD/CAM-System 4 ist dazu ausgebildet, bei Erfassung eines optischen Markers durch die intraorale Kamera ein Steuersignal zu erzeugen.

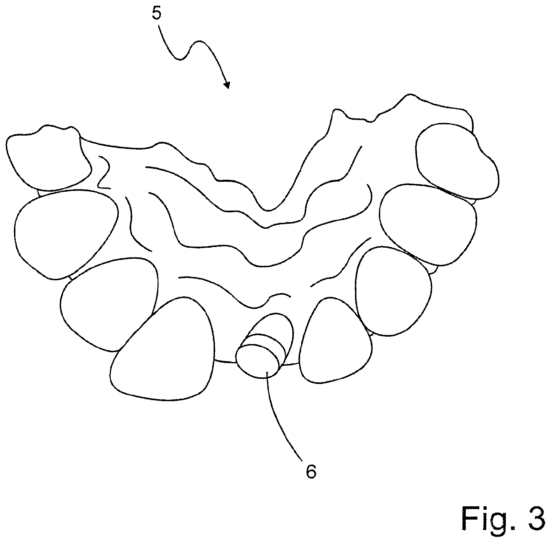

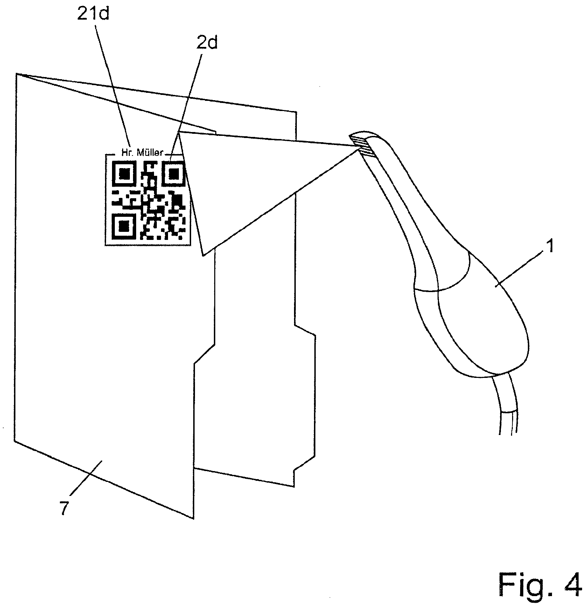

Weiterhin ist das CAD/CAM-Programm dazu eingerichtet, in einen vorgegebenen Zustand zu schalten, wenn es in einem in Fig. 3 dargestellten Kiefer 5 einen von der intraoralen Kamera 1 erfassten Scanbody 6 erkennt, der als Implantatverlängerung auf einem unter dem Scanbody 6 im Kiefer 5 befindlichen Implantat angeordnet ist. Durch die Erkennung des Scanbodys 6 wird das CAD/CAM-Programm in einen vom Zahnarzt vorgebbaren Zustand geschaltet. Dies kann beispielsweise ein Zustand sein, in dem ein aus dem Scan abgeleitetes Dreiecksnetz eine andere Auflösung erfährt als die übrigen aus dem Scan abgeleiteten Bilddaten.

Die Erkennung eines zweidimensionalen Barcodes 2a, 2b, 2c oder eines Scanbodys 6 in einem von der intraoralen 3D-Kamera 1 erfassten Bild erfolgt mittels einer Bildfilter- bzw. Analysemethode, die mittels einer Vergleichsoperation, wie beispielsweise der Erstellung eines Differenzbildes im Bildraum, einer Übertragung in den Frequenzraum, einer Suche nach ähnlichen Mustern, Frequenzen oder Signalen oder einer statistischen Methode in dem Bild nach bekannten optischen Markern sucht.

Wird in den Bilddaten ein Barcode 2a, 2b, 2c oder ein Scanbodys 6 als optischer Marker erkannt, so wird aufgrund einer in dem Computerprogramm hinterlegten Entscheidungstabelle eine dem optischen Marker zugeordnete Funktion gestartet. Wird in den Bilddaten kein optischer Marker erkannt, so werden die Bilddaten, sofern ein Scanmodus der intraoralen 3D-Kamera 1 aktiv ist, an einen in dem Computerprogramm ablaufenden Scanprozess weitergeleitet, beispielsweise einen dreidimensionalen Punktwolken- oder Tiefenbildscanprozess. Dort werden die Bilddaten für eine spätere dreidimensionale Rekonstruktion und eine Modellberechnung gespeichert.

Das CAD/CAM-Programm gemäß dieser Ausführungsform der Erfindung sowie das Verfahren zu seiner Steuerung ermöglichen somit in Verbindung mit den auf einem Träger 3 dargestellten Barcodes 2a, 2b, 2c als Vorrichtung zur Steuerung des Computerprogramms oder den auf einem Monitor 41 dargestellten Barcodes 2a, 2b, 2c die kontaktlose Steuerung einer CAD/CAM-Software, bei der der Zahnarzt die intraorale Kamera 1 nicht aus der Hand legen muss.

## Patentansprüche

1. Verfahren zur Steuerung eines Computerprogramms, wobei bei Erfassung eines optischen Markers durch einen intraoralen Scanner (1) ein Steuersignal an das Computerprogramm gesendet wird, welches das Computerprogramm in einen vorgegebenen Zustand schaltet, wobei das Computerprogramm ein CAD/CAM-Programm ist, wobei der vorgegebene Zustand ein Wechsel eines Bildfeldes, das Löschen eines Bildbereiches, eine Bildberechnung oder eine Restaurationsberechnung ist.

2. Verfahren nach Anspruch 1, **dadurch gekennzeichnet, dass** der optische Marker ein zweidimensionaler Barcode (2a, 2b, 2c) ist.

3. Verfahren nach Anspruch 1 oder 2, **dadurch gekennzeichnet, dass** der optische Marker auf einem Träger aufgebracht ist (3), der mindestens einen optischen Marker aufweist.

4. Verfahren nach Anspruch 1 oder 2, **dadurch gekennzeichnet, dass** der optische Marker auf einem Scanbody (6) aufgebracht ist oder aus einem Scanbody (6) besteht.

## Claims

1. Method for controlling a computer program, wherein a control signal which switches the computer program into a predefined state is sent to the computer program upon detection of an optical marker by an intraoral scanner (1), wherein the computer program is a CAD/CAM program, wherein the predetermined state is a change of an image field, the deletion of an image region, an image calculation or a restoration calculation.

2. Method according to Claim 1, **characterized in that** the optical marker is a two-dimensional barcode (2a, 2b, 2c).

3. Method according to Claim 1 or 2, **characterized in that** the optical marker is disposed on a support (3) which comprises at least one optical marker.

4. Method according to Claim 1 or 2, **characterized in that** the optical marker is disposed on a scan body (6) or consists of a scan body (6).

## Revendications

1. Procédé de commande d'un programme informatique, dans lequel, lors de la détection d'un marqueur optique par un scanner (1) intra-oral, un signal de commande est envoyé au programme informatique, lequel commute le programme informatique dans un état prédéterminé, le programme informatique étant un programme CAD/CAM, l'état prédéterminé étant un changement d'un champ d'image, l'effacement d'une zone d'image, un calcul d'image ou un calcul de restauration.

2. Procédé selon la revendication 1, **caractérisé en ce que** le marqueur optique est un code-barres bidimensionnel (2a, 2b, 2c).

3. Procédé selon la revendication 1 ou 2, **caractérisé en ce que** le marqueur optique est rapporté sur un support (3) qui présente au moins un marqueur optique.

4. Procédé selon la revendication 1 ou 2, **caractérisé en ce que** le marqueur optique est rapporté sur un corps de balayage (6) ou est constitué d'un corps de balayage (6) .
